(19) Europäisches Patentamt
European Patent Office
Office européen des brevets

(11) Veröffentlichungsnummer: **0 473 825 A1**

# (12) EUROPÄISCHE PATENTANMELDUNG

(21) Anmeldenummer: **90117133.0**

(22) Anmeldetag: **05.09.90**

(51) Int. Cl.5: **C07D 498/08**, A61K 31/495, //(C07D498/08,307:00,267:00)

(43) Veröffentlichungstag der Anmeldung: **11.03.92 Patentblatt 92/11**

(84) Benannte Vertragsstaaten: **AT BE CH DE GB IT LI NL**

(71) Anmelder: **TECHNOLOGICHEN KOMBINAT ZA PROMISHLENA MICROBIOLOGIA**

**BG-7200 Razgrad(BG)**

(72) Erfinder: **Konstantinova, Rumyana Georgieva**
**Stara Planina Strasse 37**
**Sofia(BG)**
Erfinder: **Evstatieva, Anka Velcheva, Dipl.-Chem.**
**Ginzi Strasse Bl. 27A**
**Sofia(BG)**
Erfinder: **Again, Ivan Alexandrov, Dipl.-Chem.**
**Ouartal Kr. Selo Bl. 195-2**
**Sofia(BG)**
Erfinder: **Dimova, Velichka Ilieva, Dr.**
**Komplex Mladost, Bl. 66-8**
**Sofia(BG)**

(74) Vertreter: **von Füner, Alexander, Dr. et al**
**Patentanwälte v. Füner, Ebbinghaus, Finck**
**Mariahilfplatz 2 & 3**
**W-8000 München 90(DE)**

## (54) Nicht-solvatierte Kristallform "A" des 3-(-4-Cinnamyl-1-piperazinyl)-iminomethylrifamyzins SV und Verfahren zu seiner Herstellung.

(57) Die nicht-solvatierte Kristallform "A" des 3-(-4-Cinnamyl-1-piperazinyl)-iminomethylrifamycins SV der formel I wird in der therapeutischen Praxis verwendet. Sie besitzt eine schnelle Resorption, hohe Serumkonzentrationen, sowie gute Stabilität und chemische Widerstandsfähigkeit. Sie stellt eine kristalline Substanz mit IR-Spektrum mit Absorptionsmaxima bei 1644, 1585, 1340, 1350, 1295 und 1290 (Dublet), 1238, 1213 und Triplet mit schwacher Intensität bei 726, 735 und 760 $cm^{-1}$, sowie auch mit charakteristischen Diffraktionsmaxima bei 9.25 und 9.50 $\theta$, am intensivsten bei 12.22 und Dublet bei 13.10 und 13.33 $\theta$ im Röntgendiffraktogramm.

Die Herstellung der nicht-solvatierten Kristallform "A" besteht darin, daß das ein 3-(-4-Cinnamyl-1-piperazinyl)-iminomethylrifamycin SV enthaltendes Rohprodukt in einem Medium aus indifferenten Lösungsmitteln, insbesondere Tetrahydrofuran oder Chloroform, umkristallisiert, in Isopropanol oder Aceton oder in einem Gemisch aus Isopropanol und Aceton bei 30 °C bis zur Siedetemperatur des entsprechenden Lösungsmittels löst und abkühlt.

EP 0 473 825 A1

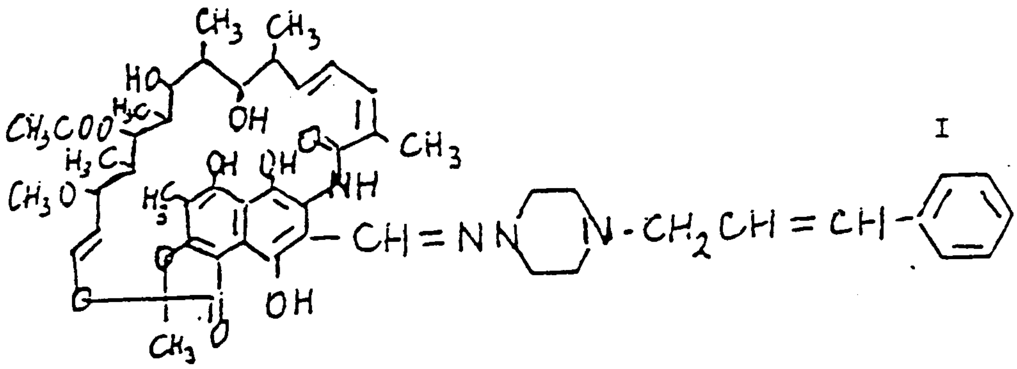

I

Die Erfindung betrifft die nicht-solvatierte, biologisch aktive Kristallform "A" des 3-(-4-Cinnamyl-1-piperazinyl)-iminomethylrifamyzins SV und ein Verfahren zu seiner Herstellung. Die Verbindung gehört zu der Gruppe der halbsynthetischen Ansamyzinen und hat eine hohe Aktivität gegenüber grampositiven (aerobischen, anaerobischen und Tuberkulosen) sowie gramnegativen Mikroorganismen in vitro und in vivo. Im Fall von generalisierter Tuberkulose von Meerschweinchen Zeigt diese Verbindung eine therapeutische Wirkung bei einer achtmal niedrigeren Dosis als die des Rifampizins (Tubozin). Es ist ein Abkömmling des Rifamycins SV mit der Formel I bekannt (BG-PS 25006/1977):

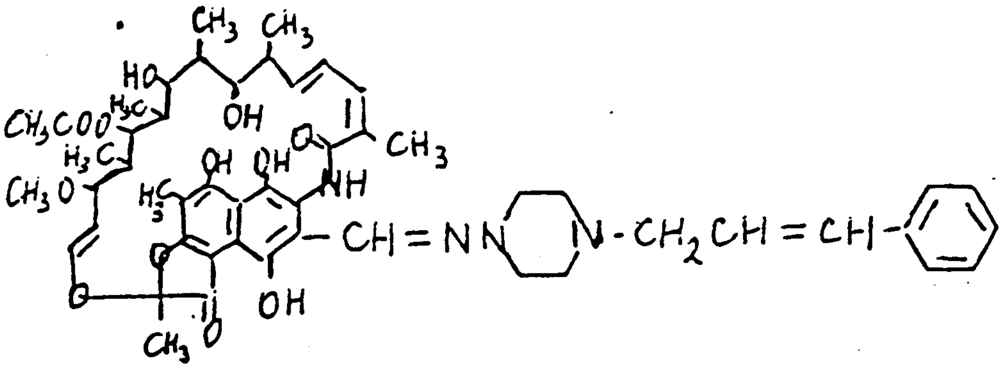

I

Die therapeutische Wirkung tritt bei einer Dosis hervor, die achtmal niedriger ist, als die des Tubozins, wobei der $LD_{50}$ von weißen Mäusen 40.000 mg/kg Gewicht beträgt, während er für Tubozin bei 1.500 mg/kg liegt. Das macht diese Verbindung zur erfolgversprechendsten aus der Gruppe der Abkömmlinge des Rifamycins SV wie in BG-PS 25006 beschrieben.

Es ist aus der Literatur bekannt, daß Tubozin in verschiedenen polymorphen Formen auftreten kann, wobei man in Abhängigkeit vom Herstellungsverfahren oder von der Reinigung nicht-solvatierte Kristallformen sowie Solvate mit verschiedenen Lösungsmitteln erhalten kann (Pelizza G. et al., Polymorphismus von Rifampizin, Farm.Ed.Sci., 1974, 7, 471-481).

Der Erfindung liegt die Aufgabe zugrunde, eine neue nichtsolvatierte Kristallform der Verbindung zu erhalten, die bei der Lagerung stabil bleibt und eine verbesserte Löslichkeit und Resorption im Organismus aufweist.

Diese Aufgabe wird durch die nicht-solvatierte Kristallform "A" des 3-(-4-Cinnamyl-1-piperazinyl)-iminomethylrifamyzin SV der Formel I

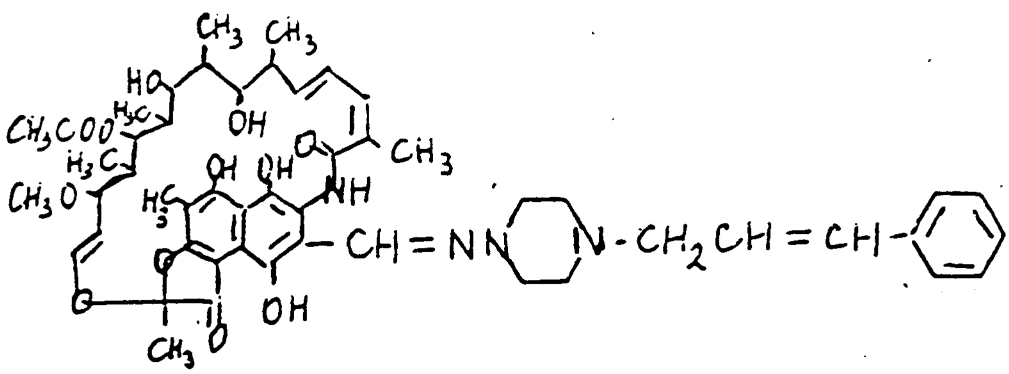

I

gelöst, die ein infrarotes Spektrum, registriert in Suspension von Nuiol mit Absorptionsmaxima bei 1644, 1585, 1340, 1350, 1295 und 1290 (Dublet), 1238, 1213 und Triplet mit schwacher Intensität bei 726, 735 und 760 $cm^{-1}$, sowie mit charakteristischen Diffraktionsmaxima bei 9.25 und 9.50 $\theta$ am intesivsten bei 12.22 und Dublet bei 13.10 und 13.33 $\theta$ im Röntgen-Diffraktogramm erreicht.

Ein Verfahren zur Herstellung der nicht-solvatierten Kristallform "A" besteht darin, daß man 3-(-4-Cinnamyl-1-piperazinyl)-iminomethylrifamycin SV in einem Medium aus indifferentem organischen Lösungsmittel, vorzugsweise Tetrahydrofuran oder Chloroform, isoliert und in Isopropanol oder Aceton, oder in einem Gemisch aus Isopropanol und Aceton bei 30 ° C bis zur Siedetemperatur des entsprechenden

Lösungsmittel löst.

Das Verhältnis der Substanz (in Gramm) zu ml Lösungsmittel hängt von der Löslichkeit der Verbindung in dem entsprechenden Lösungsmittel ab. Für Isopropanol beträgt das Verhältnis 1 g zu 70 ml und für Aceton 1 g zu 15 ml.

Erfindungsgemäß wird bevorzugt ein Gemisch aus Isopropanol und Aceton im Verhältnis 1:1 als Lösungsmittel zur Herstellung der Kristallform "A" verwendet.

Beim Lösen der Kristallform "A" in Chloroform und rascher Verdampfung des Lösungsmittels erhält man die solvatierte Kristallform "C I".

Bei der Kristallisation der Kristallform "A" in Tetrachlormethan oder Methanol erhält man die entsprechenden solvatierten Formen "C II" und "C III".

Die Vorteile der Erfindung sind folgende: die Kristallform "A" weist eine schnelle Resorption, hohe Serumkonzentrationen, eine gute chemische Stabilität, Stabilität bei normalen Lagerbedingungen auf, und kann leicht in kristallographisch reinem Zustand hergestellt werden.

## Beispiel 1

A. Herstellung von 3-(-4-Cinnamyl-1-piperazinyl)-iminomethyl-Rifamycin SV

3,6 g (0,005 Mol) 3-Formyl-Rifamycin SV werden in 30 ml Chloroform gelöst, und zu der Lösung werden unter kontinuierlichem Rühren 1,2 g (0,0055 Mol) $N^1$-Cinnamyl-$N^4$-aminopiperazin, gelöst in 10 ml Chloroform, zugefügt. Das Reaktionsgemisch wird bei Zimmertemperatur 1 Stunde gerührt und danach wird das Lösungsmittel bis zu seiner vollständigen Entfernung unter verringertem Druck abdestilliert. Zum Rest werden 30 ml Diisopropylether zugegeben. Es wird dann über Nacht im Kühlschrank stehengelassen. Der ausgefallene dunkelrote Niederschlag wird abfiltriert, mit Diisopropylether gewaschen und bei 60 °C getrocknet. Die Ausbeute beträgt 4,15 g technisches Produkt.

B. Umkristallisation des technischen Produkts unter den Bedingungen der Herstellung der nicht-solvatierten Kristallform "A"

Zu 4,15 g des technischen Produkts, das nach dem obenbeschriebenen Verfahren hergestellt wurde, werden 290 ml Isopropanol zugesetzt. Das Reaktionsprodukt wird bis zum Sieden erwärmt, die Lösung im warmen Zustand filtriert und das Filtrat unter Rühren auf 10 °C abgekühlt. Die entstandenen Kristalle werden filtriert und mit gekühltem Isopropanol gewaschen. Nach dem Trocknen werden 3,7 g der nichtsolvatierten Kristallform "A" des 3-(-4-Cinnamyl-1-piperazinyl)-iminomethyl-Rifamyzin SV mit folgenden Eigenschaften erhalten:

IR-Spektrum mit folgenden intensiven Streifen: 1644, 1585, 1340, 1350, 1295 und 1290.

Diffraktionsmaxima: bei 9.25 und 9.50 $\theta$ .

## Beispiel 2

3 g des gemäß Beispiel 1A erhaltenen technischen Produkts werden unter Bedingungen analog zu Beispiel 1B umkristallisiert, wobei statt 290 ml Isopropanol in diesem Fall 45 ml Aceton verwendet werden. Man erhält 2,1 g einer kristallinen Substanz, die gemäß dem IR-Spektrum der Form "A" entspricht.

Beim Auflösen der Kristallform "A" in Chloroform und rascher Verdampfung des Lösungsmittels erhält man eine solvatierte Kristallform "C I".

Beim Umkristallieren der Kristallform "A" mit Tetrachlormethan oder Methanol erhält man die entsprechenden solvatierten Formen "C II" und "C III".

Die verschiedenen Kristallformen wurden durch IR-Spektren aus Suspensionen in Nuiol, Röntgendiffraktion von Pulverproben und durch thermische Analyse nachgewiesen.

In den Figuren 1, 2, 3 und 4 sind die IR-Spektren der verschiedenen Kristallformen dargestellt. Es ist ersichtlich, daß sie sich in der Lage und Intensität der Absorptionsstreifen im Bereich 2000 bis 1300 $cm^{-1}$ unterscheiden. Die charakteristischen Absorptionsstreifen in den IR-Spektren, die zur Bestimmung der verschiedenen Formen benutzt werden, sind in Tabelle 1 dargestellt.

Die IR-Spektren der vier Kristallformen werden durch ein standardisiertes Verfahren, das schnell und bequem ist, mit Suspensionen in Nuiol erhalten. Die Spektren werden von einem Apparat Pye Unicam Modell 1000 erzeugt.

Aus den Röntgenogrammen (Fig. 5, 6, 7 und 8) ist ersichtlich, daß die Diffraktionsmaxima im Bereich von 5 bis 20 $\theta$ (Diffraktionswinkel) für alle Formen liegen, sich aber wesentlich nach Lage und relativer

Intensität unterscheiden, was das Bestehen von vier verschiedenen Kristallstrukturen beweist. Die oberen Werte $\theta$, die zum Unterscheiden einer der Formen von den anderen verwendet werden, sind in Tabelle 2 dargestellt. Die solvatierten Formen werden nach dem thermogravimetrischen Verfahren nach dem Gewichtsverlust nachgewiesen.

Mit den vier Kristallformen wurden harmokinetische Untersuchungen mit Versuchstieren (weißen Mäusen) zur Bestimmung ihrer Serumkonzentrationen durchgeführt. Den Versuchstieren wurden per os eine 10%igen wäßrigen Lösung einer Tween 80 Suspension der vier Kristallformen in Dosen von 40 mg/kg verabreicht. Die Blutproben wurden 15, 30 und 60 Minuten nach der Einnahme entnommen. Die Serumkonzentrationen der verschiedenen Kristallformen nach Minuten werden nach dem Zentrifugieren nach dem Diffusionsverfahren in einem Medium aus Agar nach Rendal mit dem Test-Stamm Microkokkus Luteus ATSS 9341 gegenüber Tubozin als Vergleich bestimmt. Man kann in Tabelle 3 die erhaltenen Ergebnisse vergleichen.

Aus dieser Tabelle ist ersichtlich, daß die Kristallform "A" die schnellste Resorption aufweist und Serumkonzentrationen erreicht, die mit denen für Tubozin vergleichbar sind. Es ist auch hervorzuheben, daß die Form "A" eine therapeutische Wirkung noch bei einer achtmal niedrigeren Dosis als bei Tubozin besitzt.

Die Untersuchungen zur Stabilität der Form "A" werden mit einer Probe im Aufschüttungszustand, die in einem Ofen bei 50°C gehalten wurde, und mit einer Probe, die dem Sonnenlicht bei 25°C ausgesetzt wurde, durchgeführt. Nach 6 und 12 Wochen wurden die Proben auf offensichtliche visuelle oder chemische Veränderungen geprüft. Es wurde festgestellt, daß die Proben eine gute chemische Stabilität aufweisen. Aus den durchgeführten Untersuchungen mit den verschiedenen Kristallformen ist ersichtlich, daß die Verbindung mit der Form "A" folgende Vorteile hat:

- die schnellste Resorption und höhere Serumkonzentrationen,
- Stabilität bei normalen Lagerbedingungen
- leichte und einfache Herstellung in reinem kristallografischen Zustand.

Tabelle 1

Charakteristische Absorptionsstreifen in IR-Spektren der verschiedenen Kristallformen, $cm^{-1}$

| nicht solvatierte Form "A" | Solvat mit $CHCl_3$ C I | Solvat mit $CCl_4$ C II | Solvat mit $CH_3OH$ C III |
|---|---|---|---|
| 1 | 2 | 3 | 4 |
| 3420 | 3580 | 3360 | 3620 |
| 1644 | 3450 | 3200 | 3280 |
| 1555 | | 1703 | 1720 |
| 1460 | 1665 breiter Triplet | 1385 | |
| | 1650 | 1300 | 1645 breiter |
| 1340 Dublet | 1625 | 1180 | 1630 Triplet |
| 1350 | | 1090 | 1600 |
| | 1420 | 1030 | |
| 1295 Dublet | 1335 | | 1450 |
| 1290 | | 940 | 1315 |

| 1 | 2 | 3 | 4 |
|---|---|---|---|
| | 1000 | 915 Triplet | 1100 |
| | Triplet | | |
| 1238 | 980 | 900 | 1065 - arm |
| 1213 | 953 | | 1050 |
| | | 770 | 1015 |
| 760 | 920 | 755 - arm | |
| | Dublet | | |
| 735 Triplet | 895 | 715 - arm | 980 |
| 726 | | 700 | 955 Triplet |
| | 775 | | 905 |
| 700 | 748 Triplet | | |
| Dublet | | | |
| 690 | 730 | | 785 |
| | 710 | | 760 Triplet |
| | Dublet | | |
| | 700 | | 753 |
| | | | 715 Duplet |
| | | | 697 |

Tabelle 2

Charakteristische Diffraktionsmaxima in Röntgenogrammen der verschiedenen Kristallformen, in Diffraktionswinkeln 0

| Nicht solvatierte Kristallform "A" | Solvat mit $CHCl_3$ C II | Solvat mit $CCL_4$ C III | Solvat mit $CH_3OH$ C IV |
|---|---|---|---|
| 5,4 | 6,3 | | 5,5 |
| 5,6 | 6,8 | | |
| 6,1 | 8,8 am intensiv- | 9,3 inten- | 7,2 Triplet |
| 9,2 am intensivsten | sten | 9,6 sives | 7,5 mit höchster |
| | | 9,8 Triplet | 7,7 Intensität |
| 9,5 Dublet | 10,8 | | |
| | 11,1 | 10,5 | 8,0 |
| | | 10,8 Triplet | 8,1 |
| 10,1 | | 11,0 | |
| 10,6 | | | 9,0 |
| Dublet | | 11,4 | 10,4 |
| 10,7 | | 12,4 | |
| 12,2 | | | 11,0 |
| 13,1 | | | 13,3 Triplet |
| Dublet | | | 11,6 |
| 13,3 | | | 13,1 |
| | | | Dublet |
| | | | 13,4 |
| 14,8 | | | 14,3 |
| Dublet | | | |
| 15,1 | | | |

Tabelle 3

Serumkonzentrationen der verschiedenen Kristallformen bei Versuchstieren nach peroraler Verabreichung der Dosis 40 mg/kg Gewicht, mkg/ml

| Kristallform | Minuten nach der Verabreichung | | |
|---|---|---|---|
| | 15' | 30' | 60' |
| C I | 6,2 | 8,6 | 8,8 |
| C II | 2,4 | 4,2 | 3,6 |
| C III | 4,0 | 6,8 | 7,6 |
| C IV | 1,8 | 3,6 | 3,2 |
| Tubozin | 6,8 | 8,8 | 10,10 |

## Patentansprüche

1. Nicht solvatierte Kristallform "A" des 3-(-4-Cinnamyl-1-piperazinyl)-iminomethylrifamycin SV der Formel I:

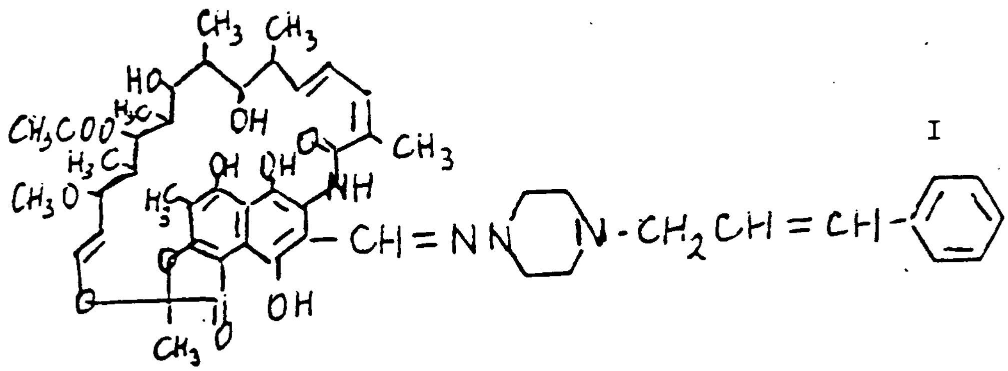

I

mit einem IR-Spektrum, registriert in einer Suspension von Nuiol mit Absorptionsmaxima bei 1644, 1585, 1340, 1350, 1295 und 1290 (Dublet), 1238, 1213 und Triplet mit schwacher Intensität bei 726, 735 und 760 $cm^{-1}$ sowie mit charakteristischen Diffraktionsmaxima bei 9.25 und 9.50 $\theta$, am intensivsten bei 12.22 und Dublet bei 13.10 und 13.33 $\theta$ im Röntgendiffraktogramm.

2. Verfahren zur Herstellung der nicht solvatierten Kristallform "A" gemaß Anspruch 1, **dadurch gekennzeichnet,** daß ein Rohprodukt von 3-(-4-Cinnamyl-1-piperazinyl)-iminomethylrifamyzin SV in einem Medium aus indifferenten Lösungsmitteln, insbesondere Tetrahydrofuran oder Chloroform, umkristallisiert und danach in Isopropanol oder Aceton oder in einem Gemisch aus Isopropanol und Aceton bei 30 ° C bis zur Siedetemperatur des entsprechenden Lösungsmittels löst und abkühlt.

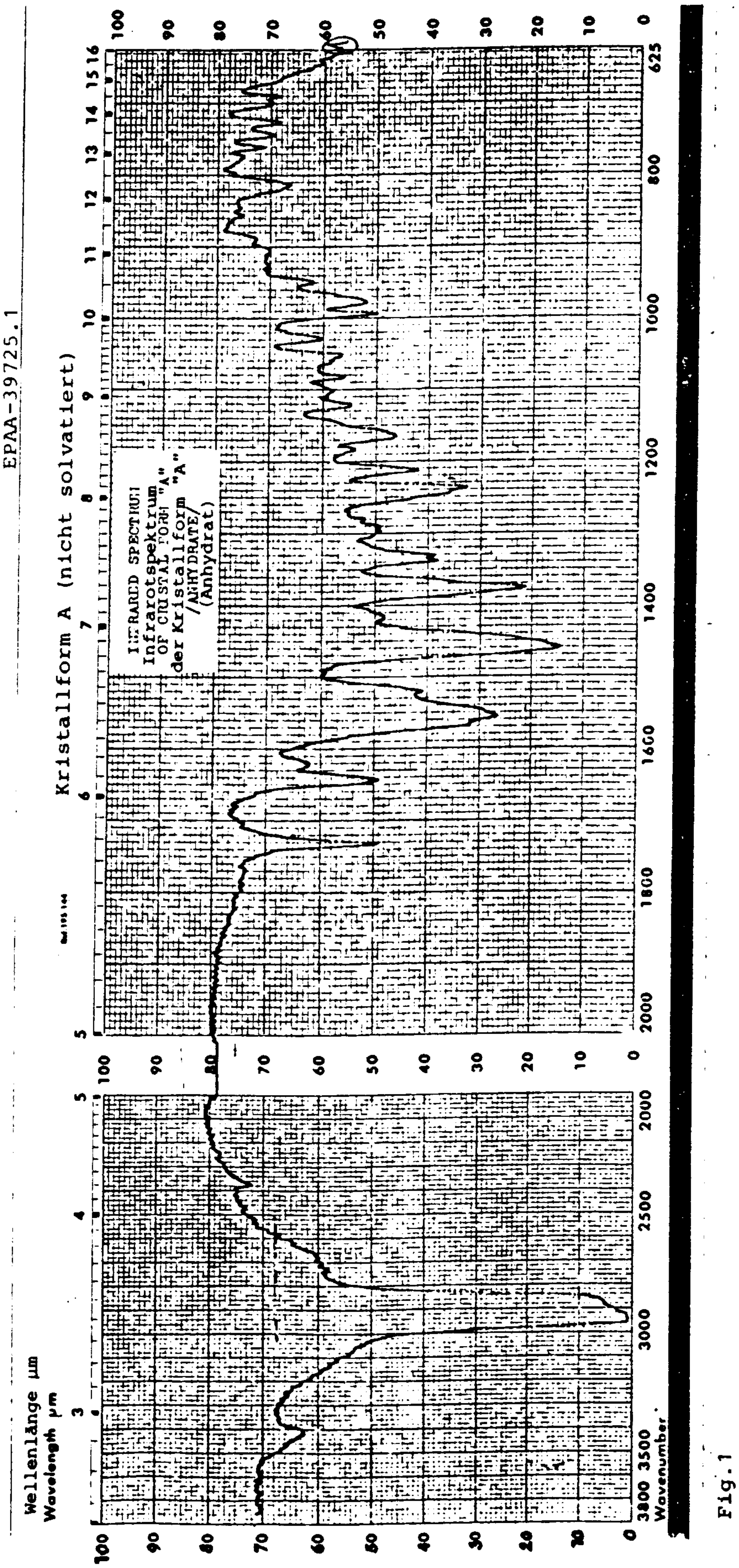
EPAA-39725.1
Kristallform A (nicht solvatiert)
INFRARED SPECTRUM
Infrarotspektrum
OF CRYSTAL FORM "A"
der Kristallform "A"
/ANHYDRATE/
(Anhydrat)
Wellenlänge µm
Wavelength µm
Wavenumber

Fig.1

EPAA-39725.1

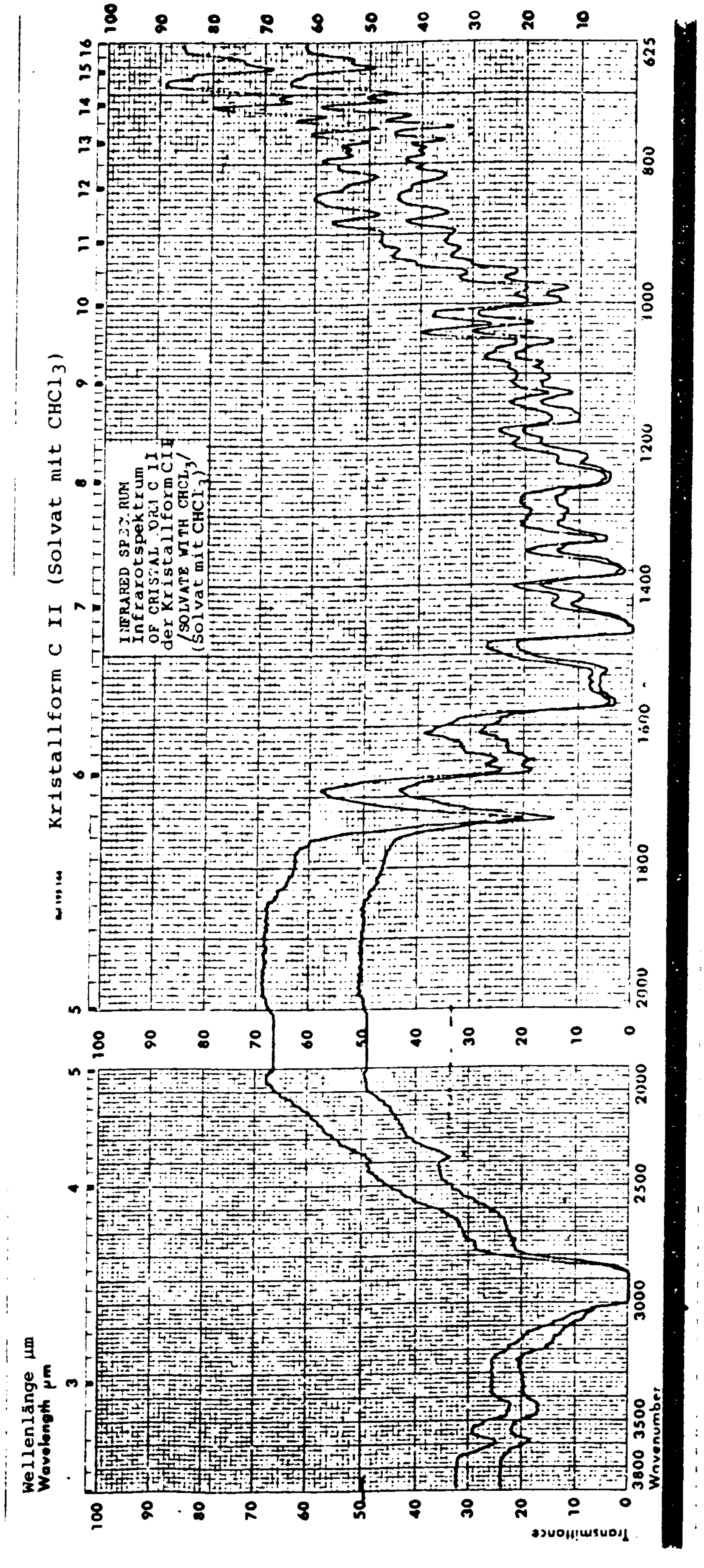


Fig.2

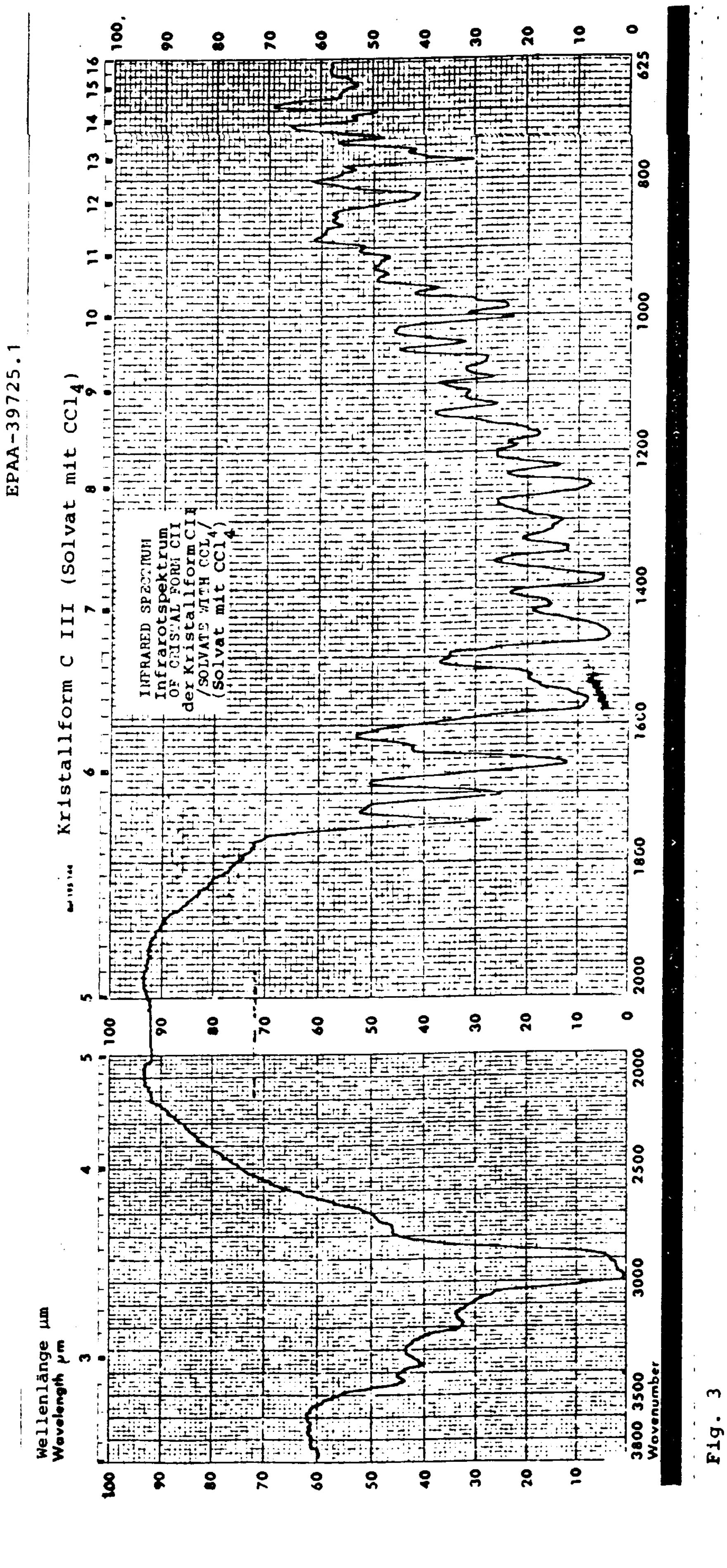
EPAA-39725.1
Kristallform C III (Solvat mit $CCl_4$)
Infrarotspektrum
der Kristallform CII
(Solvat mit $CCl_4$)
Wellenlänge µm
Wavelength µm
Wavenumber

Fig. 3

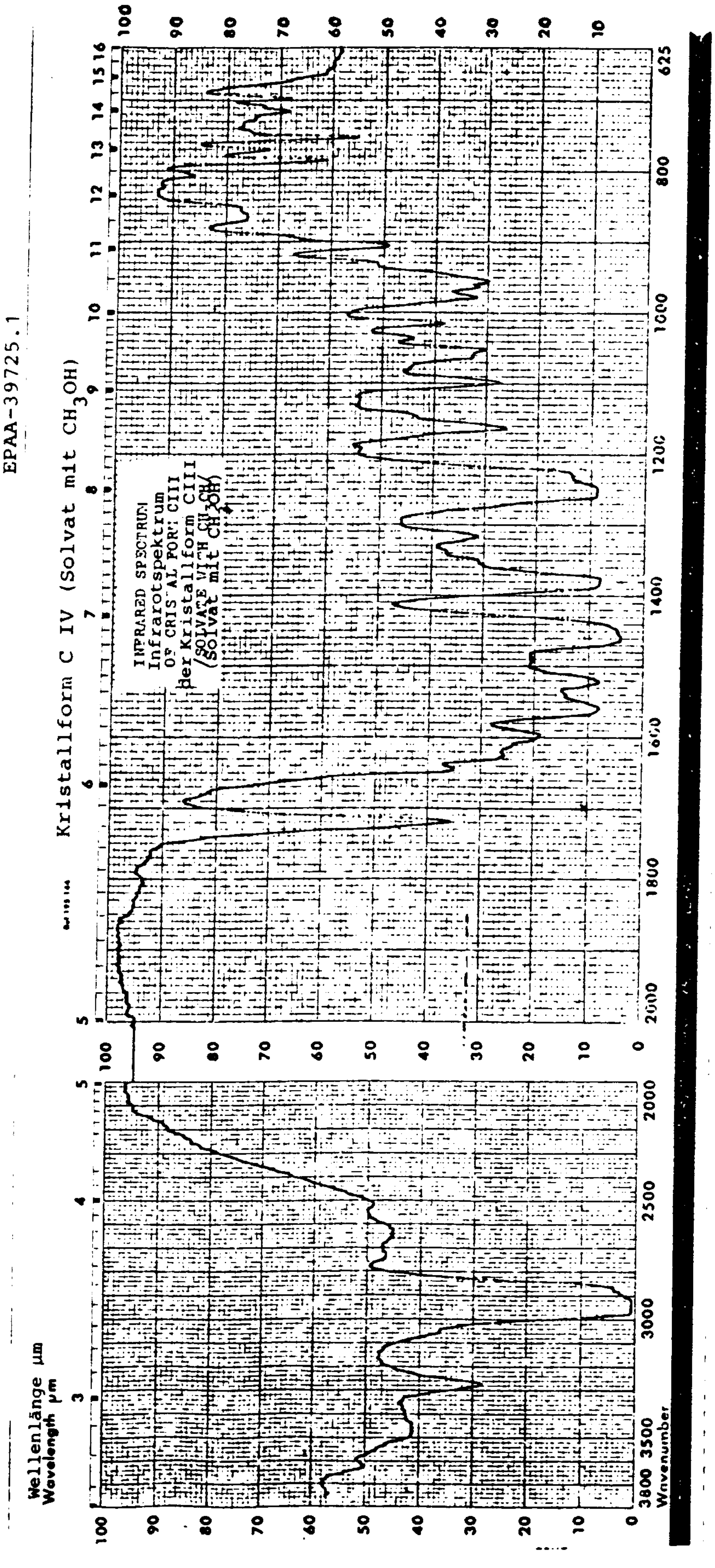
EPAA-39725.1
Kristallform C IV (Solvat mit CH3OH)
INFRARED SPECTRUM
Infrarotspektrum
der Kristallform CIII
Wellenlänge µm
Wavelength µm
Wavenumber

Fig. 4

Fig. 5

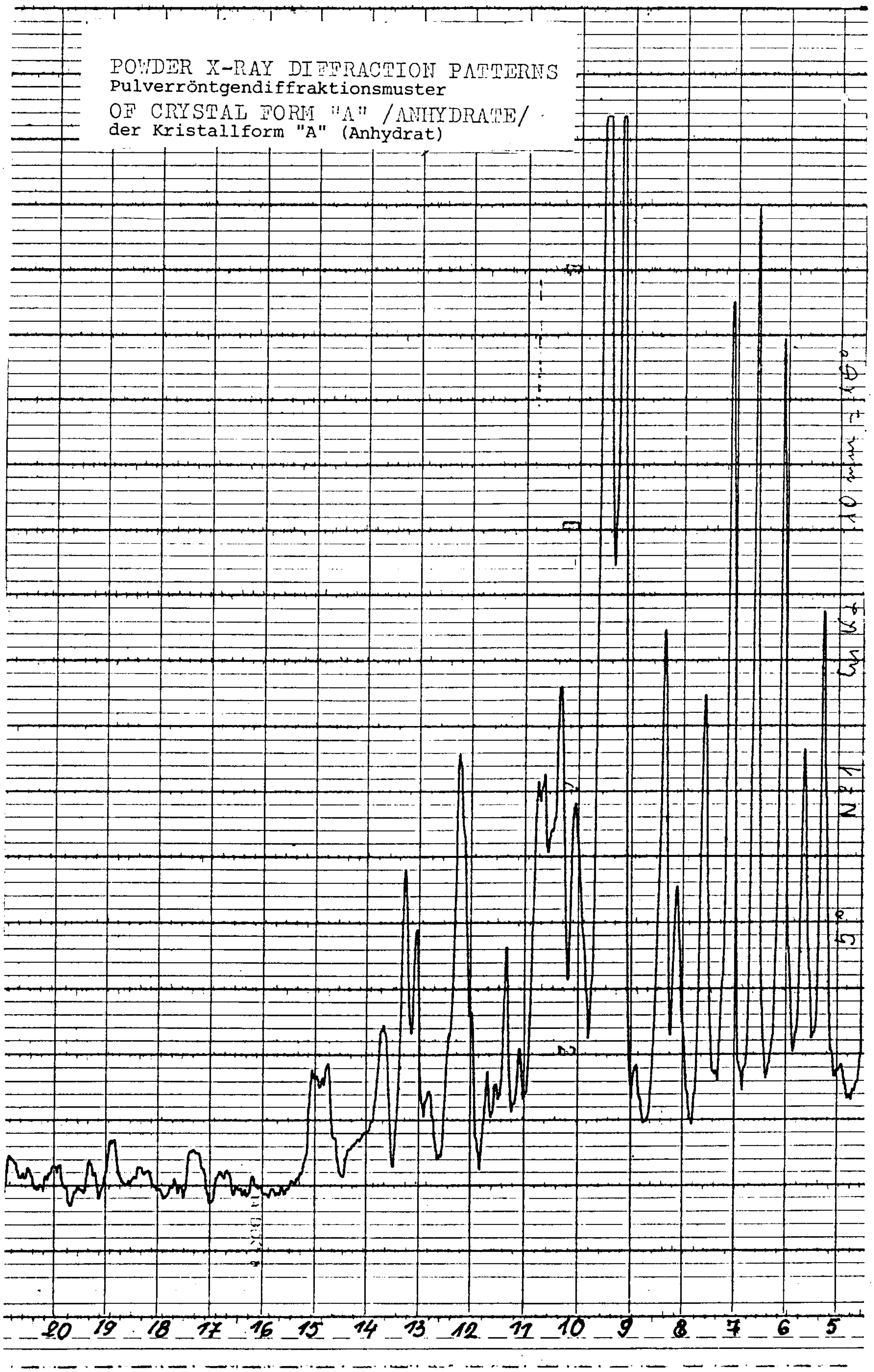
POWDER X-RAY DIFFRACTION PATTERNS
Pulverröntgendiffraktionsmuster
OF CRYSTAL FORM "A" /ANHYDRATE/
der Kristallform "A" (Anhydrat)
20 19 18 17 16 15 14 13 12 11 10 9 8 7 6 5

Fig. 6

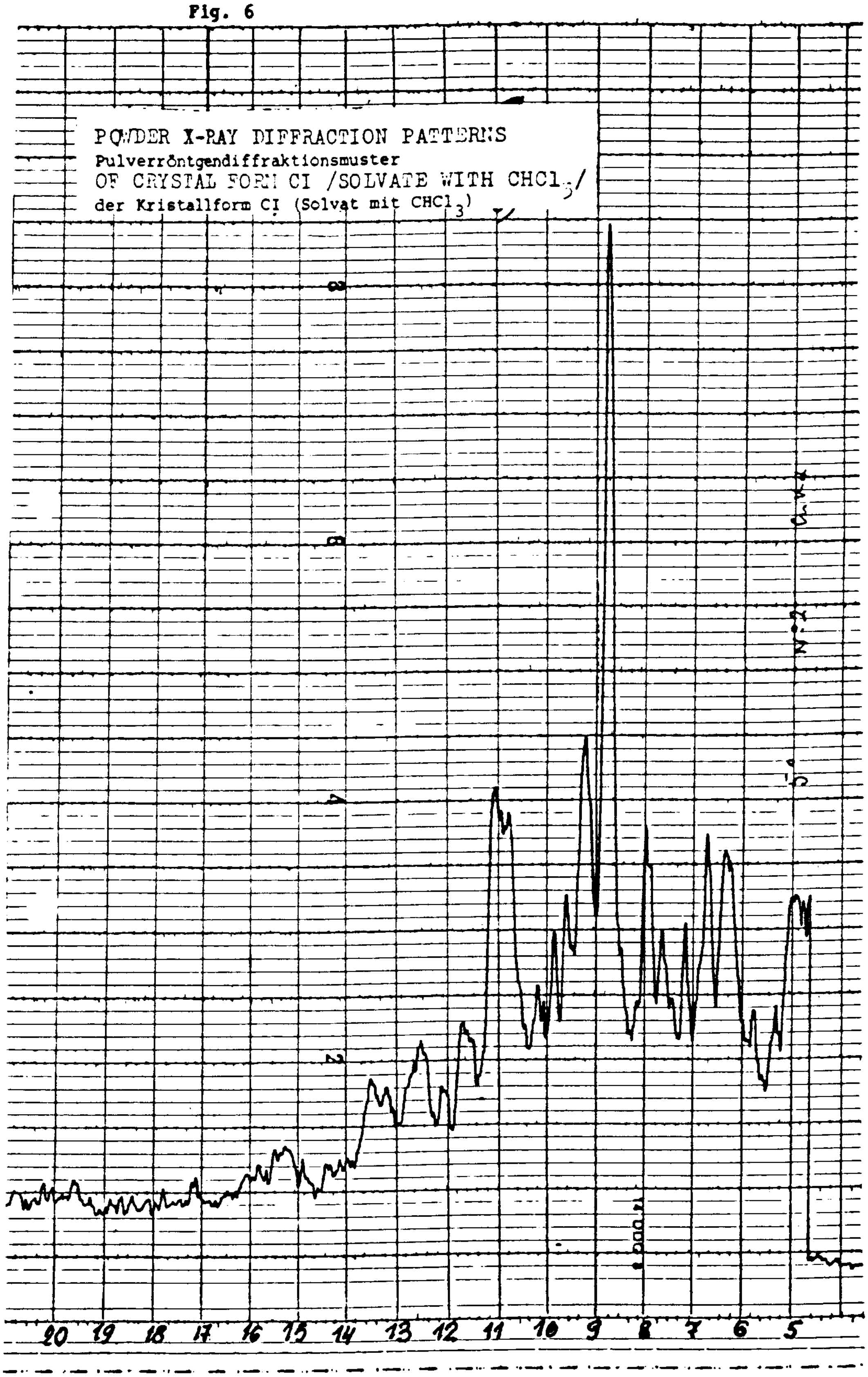
POWDER X-RAY DIFFRACTION PATTERNS
Pulverröntgendiffraktionsmuster
OF CRYSTAL FORM CI /SOLVATE WITH $CHCl_3$/
der Kristallform CI (Solvat mit $CHCl_3$)
20 19 18 17 16 15 14 13 12 11 10 9 8 7 6 5

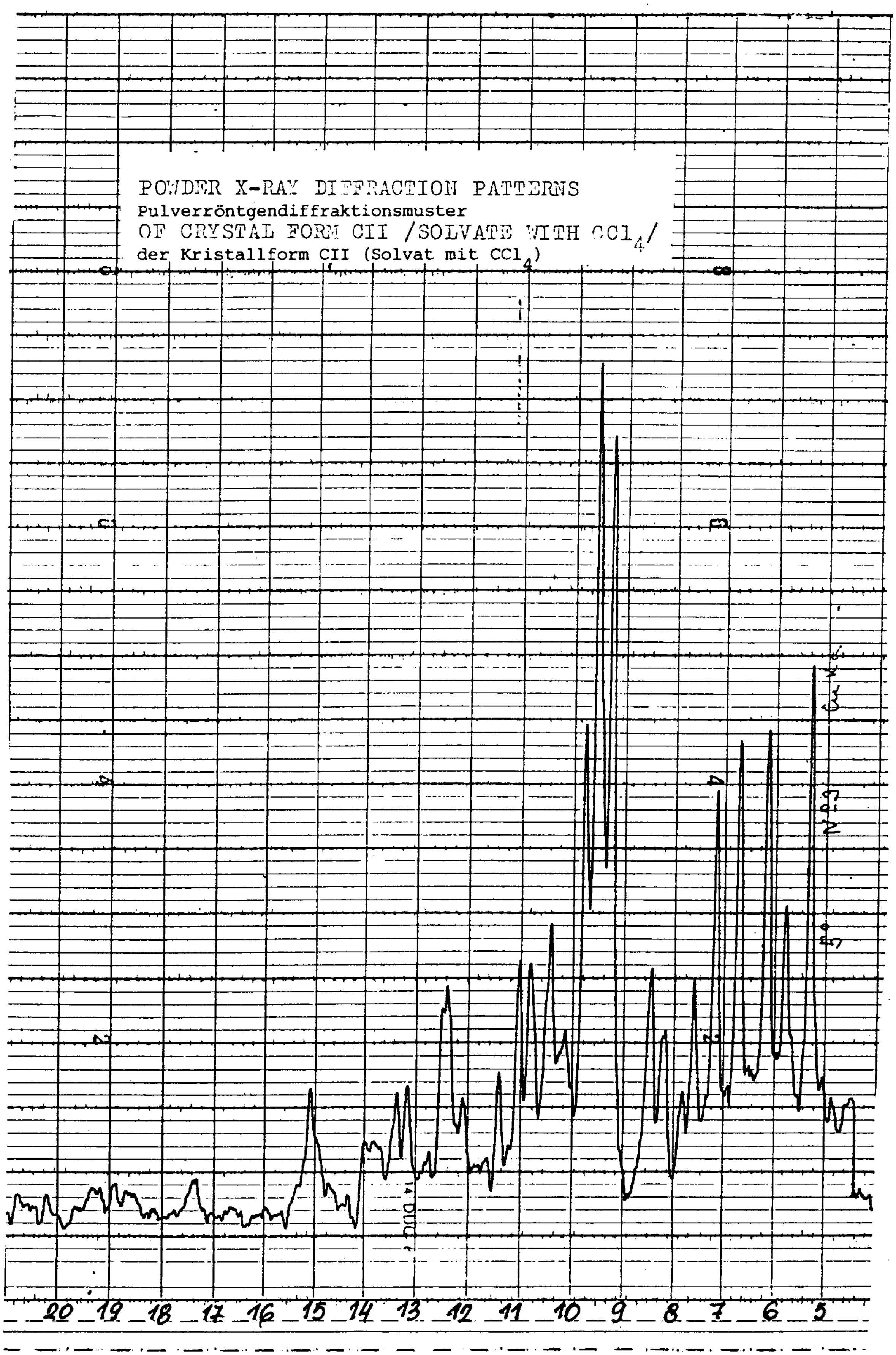
POWDER X-RAY DIFFRACTION PATTERNS
Pulverröntgendiffraktionsmuster
OF CRYSTAL FORM CII /SOLVATE WITH $CCl_4$/
der Kristallform CII (Solvat mit $CCl_4$)
20 19 18 17 16 15 14 13 12 11 10 9 8 7 6 5

Fig. 7

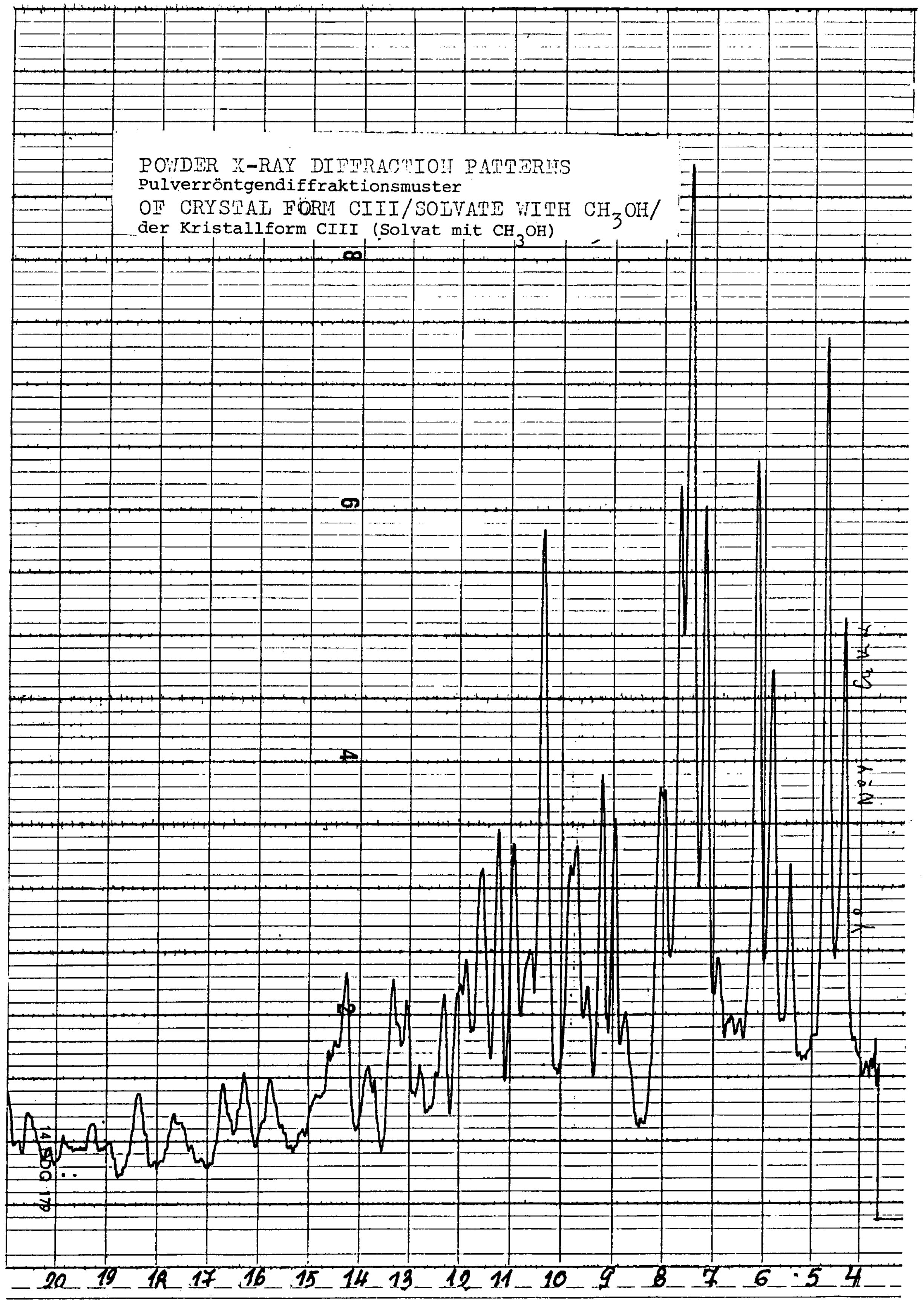
POWDER X-RAY DIFFRACTION PATTERNS
Pulverröntgendiffraktionsmuster
OF CRYSTAL FORM CIII/SOLVATE WITH $CH_3OH$/
der Kristallform CIII (Solvat mit $CH_3OH$)
20 19 18 17 16 15 14 13 12 11 10 9 8 7 6 5 4

Fig. 8

Europäisches Patentamt

# EUROPÄISCHER RECHERCHENBERICHT

Nummer der Anmeldung

**EP 90 11 7133**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-2 816 274 (DSO PHARMACHIM)<br>* Patentanspruch 1; Seite 5; Seite 6, Tabelle 1, Nr. 2 *<br>– – – | 1,2 | C 07 D 498/08<br>A 61 K 31/495 //<br>(C 07 D 498/08<br>C 07 D 307:00<br>C 07 D 267:00 ) |
| A | IL FARMACO EDIZIONE SCIENTIFICA, Band 32, Nr. 7, Juli 1977, Seiten 471-481, Societa Chimica Italiana, Rome, IT; G. PELIZZA et al.: "Polymorphism of rifampicin"<br>* Seiten 471,472 *<br>– – – – – | 1 | |

| RECHERCHIERTE SACHGEBIETE (Int. Cl.5) |
|---|
| C 07 D 498/00<br>A 61 K 31/00 |

Der vorliegende Recherchenbericht wurde für alle Patentansprüche erstellt

| Recherchenort | Abschlußdatum der Recherche | Prüfer |
|---|---|---|
| Den Haag | 19 April 91 | VOYIAZOGLOU D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument